# EUROPEAN PATENT SPECIFICATION

(11) **EP 0 973 582 B1**
(45) Date of publication and mention of the grant of the patent: **11.05.2005**
(21) Application number: 97954585.2
(22) Date of filing: 16.12.1997
(51) Int. Cl.: A61N 1/39

(54) **ELECTROTHERAPY CURRENT WAVEFORM**
STROMWELLENFORM FÜR ELEKTROTHERAPY
FORME DE COURANT POUR ELECTROTHERAPIE

(30) Priority: 18.12.1996 US 769046; 18.12.1996 US 769778; 18.12.1996 US 769773; 18.12.1996 US 769777; 18.12.1996 US 769045; 18.12.1996 US 769776; 13.08.1997 US 910757
(43) Date of publication of application: 26.01.2000
(60) Divisional application: 05075510.7
(73) Proprietor: ZMD CORPORATION, Wilmington, DE 19810 (US)
(72) Inventor: AYATI, Shervin, Sudbury, MA 01776 (US); LOPIN, Michael, L., Newton, MA 01776 (US)
(74) Representative: Hitchcock, Esmond Antony
(86) International application number: PCT/US1997/023400
(87) International publication number: WO 1998/026841

(56) References cited:
- EP-A- 0 546 666
- US-A- 4 823 796
- US-A- 5 350 403
- US-A- 5 391 186
- US-A- 5 431 687
- US-A- 5 433 732
- US-A- 5 507 781
- US-A- 5 531 764

## Description

This invention relates to electrotherapy circuits and more particularly relates to external defibrillators that apply defibrillation shocks to a patient's heart through electrodes placed externally on the patient's body or externally on the patient's heart during surgery.

Normally, electro-chemical activity within a human heart causes the organ's muscle fibers to contract and relax in a synchronized manner. This synchronized action of the heart's musculature results in the effective pumping of blood from the ventricles to the body's vital organs. In the case of ventricular fibrillation (VF), however, abnormal electrical activity within the heart causes the individual muscle fibers to contract in an unsynchronized and chaotic way. As a result of this loss of synchronization, the heart loses its ability to effectively pump blood.

Defibrillators produce a large current pulse that disrupts the chaotic electrical activity of the heart associated with ventricular fibrillation and provide the heart's electro-chemical system with the opportunity to re-synchronize itself. Once organized electrical activity is restored, synchronized muscle contractions usually follow, leading to the restoration of effective cardiac pumping.

The current required for effective defibrillation is dependent upon the particular shape of the current waveform, including its amplitude, duration, shape (i.e., sine, damped sine, square, exponential decay), and whether the current waveform has a single polarity (monophasic) or has both positive and negative polarity (biphasic). It has been suggested that large defibrillation currents may cause damage to cardiac tissue, however.

It is known to construct an external defibrillator that can sense patient impedance and can set the durations of the first and second phases of a biphasic waveform as a function of the patient impedance. An example of such a defibrillator is described in PCT Patent Publication No. WO 95/05215. Fain et al., U.S. Pat. No. 5,230,336 discloses a method of setting pulse widths of monophasic and biphasic defibrillation waveforms based on measured patient impedance. Kerber et al., "Advance Prediction of Transthoracic Impedance in Human Defibrillation and Cardioversion: Importance of Impedance in Determining the Success of Low-Energy Shocks," 1984, discloses a method of selecting the energy of defibrillation shocks based on patient impedance measured using a high-frequency signal.

It is also known to construct a defibrillator with a safety resistor in the defibrillation path (PCT Patent Publication No. WO 95/05215). Before application of a defibrillation waveform to a patient, a test pulse is passed through the safety resistor while a current sensor monitors the current. If the sensed current is less than a safety threshold representative of a short circuit, the safety resistor is removed and the defibrillation waveform is applied to the patient.

It is known, in an implantable defibrillator, to use a biphasic waveform having a first phase consisting of multiple truncated decaying exponentials that form a sawtooth approximation of a rectilinear shape (Kroll, U.S. Patent No. 5,199,429). This is accomplished by charging a set of energy storage capacitors and then successively allowing individual capacitors to discharge during the first phase, thereby creating the sawtooth pattern in the output current of the circuit. A more recent patent, Kroll, U.S. Patent No. 5,514,160, describes a biphasic waveform, in an implantable defibrillator, having a rectilinear-shaped first phase created by placing a MOSFET current limiter in the defibrillation path. This patent states that the grossly non-linear current limiter looks like a small and declining resistance to the capacitor. Also, Schuder et al., "Transthoracic Ventricular Defibrillation in the 100 kg Calf with Symmetrical One-Cycle Bidirectional Rectangular Wave Stimuli" describes the use of biphasic waveforms having rectilinear first and second phases to reverse ventricular fibrillation in calves. Stroetmann et al., U.S. Patent No. 5,350,403, discloses a waveform having a sawtooth ripple that is formed by periodically interrupting a noncontinuous discharge of a charging circuit.

U.S. Patent No. 5,531,764 discloses an implantable defibrillator having programmable shock waveforms and paths where each successive waveform may be of a different shape and form, and delivered to and through an area of the human heart in a desired sequence. The shock waveforms can be delivered independently through certain areas of the heart or through different areas of the heart to the can electrode or to a patch electrode at a computed common time. Alternatively, a first shock waveform or set of shock waveforms can be delivered through one or more areas of the heart followed by a delivery of time sequenced delayed shock waveform or forms through specific areas of the heart to the can electrode or patch electrode.

U.S. Patent No. 5,391,186 discloses an implantable cardioverter defibrillator utilizing an interrupted discharged output in order to control the output voltage waveform shape.

The present invention is directed at an electrotherapy circuit for administering to a patient a current waveform, comprising a charge storage device, at least two discharge electrodes connected by electrical circuitry to opposite poles of the charge storage device, and a control circuit connected to the charge storage device. According to the invention the circuit controls a continuous discharge of the charge storage device through the electrodes so as to produce one phase of a current waveform wherein the difference between the peak current of the phase and the lowest current of the phase is less than one-third of the peak current of the phase, followed by another phase of the current waveform having a polarity opposite to that of the one phase and a duration less than that of the one phase. In preferred embodiments, the difference between the peak current of the one phase and the lowest amount of the phase is less than one fourth, or more preferably less than one fifth of the peak current. The other phase of the current waveform is typically in the form of a truncated exponential.

In preferred embodiments of the invention at least one of the phases of the current waveform comprises a ripple. The phase of the waveform having the ripple is typically a substantially rectilinear positive phase of a biphasic waveform. We believe that the use of a waveform having a substantially rectilinear positive phase tends to minimize the threshold of average current required for effective defibrillation, and tends to avoid damaging the patient's tissue even if the total energy applied to the patient is relatively high.

In order to provide a lower threshold of average current required for effective defibrillation and minimize the possibility of damaging the patient's tissue, the height (upward jump) of the ripple may be restricted to less than one-third of the height of the peak current of the phase, and more preferably less than one-fourth or one-fifth of the height of the peak current of the phase. We believe the duration of the phase that includes the ripple should be between 50 and 70 percent (e.g., three-fifths of five-eights) of the combined duration of the phase that includes the ripple and the other phase. We also believe that any sawtooth ripple in either phase of the waveform should preferably have a height less than about one-quarter of the average height of the phase, and more preferably less than about one-sixth of the average height of the phase, in order to further minimize the threshold of average current required for effective defibrillation and the possibility of damaging the patient's tissue.

The electrotherapy circuit of the invention will normally include a resistive circuit connected between the charge storage device and one of the electrodes, and a control circuit. The control circuit is connected to the resistive circuit and controls the resistance of the resistive circuit during discharge of the charge storage device so as to shape a current waveform.produced between the discharge electrodes.

The resistive circuit can include a set of resistors connected together in series.

In preferred embodiments the control circuit decides, based on the patient impedance sensed during an initial sensing pulse portion of the discharge of the charge storage device, how many (if any) resistors to include in the defibrillation path at the beginning of a therapeutic discharge portion of the discharge of the charge storage device (e.g., at the beginning of a biphasic defibrillation waveform). This may mean, depending on the sensed patient impedance, that the current level steps up from the sensing pulse to the beginning of the biphasic defibrillation waveform. Once the biphasic defibrillation waveform begins, the resistors that are present in the defibrillation path are successively shorted out, thereby creating a sawtooth approximation to a rectilinear shape in the output current (output decays and then jumps up every time a resistor is shorted out).

The invention provides an improved, low-cost way of creating a biphasic waveform having a rectilinear first phase. Resistors are relatively inexpensive as compared with capacitors, and a total of N steps in resistance values can be obtained with log₂N resistors, as opposed to N capacitors, simply by connecting the resistors in series in a binary sequence (1-2-4-etc.). Because resistors are used instead of capacitors, no circuitry is required to equalize voltages on capacitors upon recharge or to prevent reversal of voltages on capacitors.

Certain embodiments include a variable resistor stage that tends to smooth out the ripple pattern. The variable resistor stage is a circuit that is reset to its maximum resistance value every time one of the fixed-value resistors is shorted out and then decreases to zero over the time interval before the next resistance step reduction.

Another advantage of the invention is that the resistors in the defibrillation path inherently protect against possible short circuits.

According to another aspect of the invention, the electrotherapy circuit includes a variable impedance connected between the charge storage device and one of the electrodes, a sensor that senses a patient-dependent electrical parameter (such as a patient impedance sensor), and a control circuit. The control circuit is connected to the sensor and the variable impedance and controls the variable impedance during discharge of the charge storage device based on a patient-dependent electrical parameter (such as the patient impedance) sensed by the sensor.

According to another aspect of the invention, the discharge is controlled in a manner so as to reduce the dependence of peak discharge current on the electrical parameter for a given amount of charge stored by the charge storage device.

By controlling the discharge of the charge storage device based on the sensed patient impedance, it is possible to limit the difference in the peak current that passes through a low-impedance patient as compared with a high-impedance patient. Thus, the current is made more constant over a range of patient impedances, and the electrotherapy circuit provides effective defibrillation while maintaining controlled current levels to reduce any possibility of damage to heart, skin, and muscle tissue.

According to another aspect of the invention, the discharge of the charge storage device includes a current waveform having a sensing pulse portion, with insufficient energy for performing therapy, during which the sensor senses the patient-dependent electrical parameter, and a therapeutic discharge portion, with sufficient energy for performing therapy, having an initial discharge current controlled by the control circuit based on the patient-dependent electrical parameter as sensed by the sensor. The sensing pulse portion has a discharge current that is at least about one-third of the initial discharge current of the therapeutic discharge portion.

The impedance of a patient when a large direct current is passing through the patient is different from the impedance of the patient when a small current is passing through the patient or when an alternating current is passing through the patient. We believe that the current level of the sensing portion should always be at least one-third, and more preferably one-half, of the current level at the beginning of the therapeutic discharge portion in order to ensure detection of a patient impedance that is similar to the impedance of the patient during the therapeutic discharge portion.

According to another aspect of the invention, the discharge of the charge storage device occurs without recharging of the charge storage device between the sensing pulse portion and the therapeutic discharge portion of the current waveform.

Thus, it is possible to apply paddles to the chest of the patient (or apply hand-held spoons directly to the patient's heart during open heart surgery) and immediately discharge the sensing pulse and then discharge the biphasic defibrillation waveform immediately after the sensing pulse. This is particularly important because the patient (or the patient's heart) may move and because it is difficult for a practitioner to apply a constant force to the patient's skin (or the patient's heart).

Numerous other features, objects, and advantages of the invention will become apparent from the following detailed description of embodiments thereof, when read in connection with the accompanying drawings.
Fig. 1 is a diagram of a current waveform produced by a electrotherapy circuit according to the invention.
Fig. 2 is a diagram of the key elements of electrotherapy circuit according to the invention.
Fig. 3 is a schematic diagram of the series-connected resistor circuit shown in the electrotherapy circuit of Fig. 2.
Fig. 4 is a schematic diagram of the H-bridge circuit shown in the electrotherapy circuit of Fig. 2.
Fig. 5 is a schematic diagram of the variable resistor shown in the electrotherapy circuit of Fig. 2.
Figs. 6-9 are diagrams of current waveforms produced by an electrotherapy circuit according to the invention based on different measured patient impedances.
Figs. 10A and 10B is a set of schedules of the resistance values used for generating the waveforms shown in Figs. 6-10B.
Fig. 11 is a table of waveform parameters for various patient impedances in a "normal" mode of operation and a "high-energy" mode of operation of an electrotherapy circuit according to the invention.

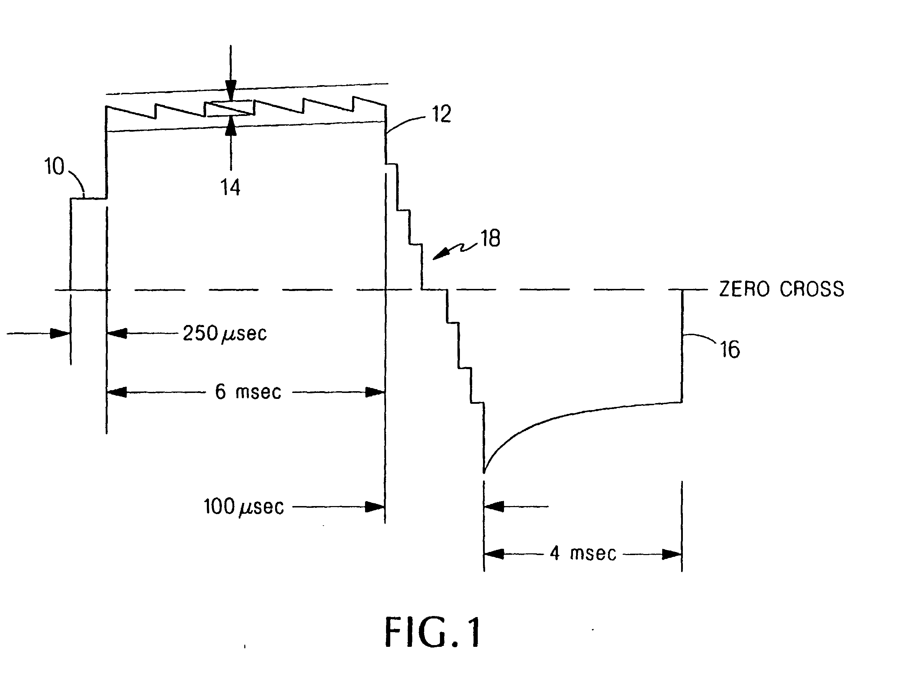

With reference to Fig. 1, in operation of an external defibrillator according to the invention, the biphasic current waveform begins with an initial "sensing pulse" 10, which has insufficient energy for performing therapy. The sensing pulse is integral with, i.e., immediately followed by, a biphasic defibrillation waveform having sufficient energy for defibrillating the patient's heart. The biphasic defibrillation waveform includes a six-millisecond, generally rectilinear positive phase 12 having a sawtooth ripple 14, which is in turn followed by a four millisecond negative phase 16 that decays exponentially until the waveform is truncated. As used herein, the term "rectilinear" means having a straight line, regardless of whether the straight line is flat or slightly tilted. The current waveform decreases through a series of steps 18 from the end of the positive phase to the beginning of negative phase, one of the steps being at the zero crossing. Note that for purposes of clarity this 0.1-millisecond transition is not drawn to scale in Fig. 1; if drawn to scale the duration of this transition would be much shorter than shown in Fig. 1.

We believe that a biphasic defibrillation waveform having a positive rectilinear pulse of 6 milliseconds duration followed by 0.1-millisecond transition and a 4 millisecond negative pulse having an initial amplitude equal to the final amplitude of the positive pulse is an especially effective waveform for defibrillation. The negative pulse does not need to be rectilinear.

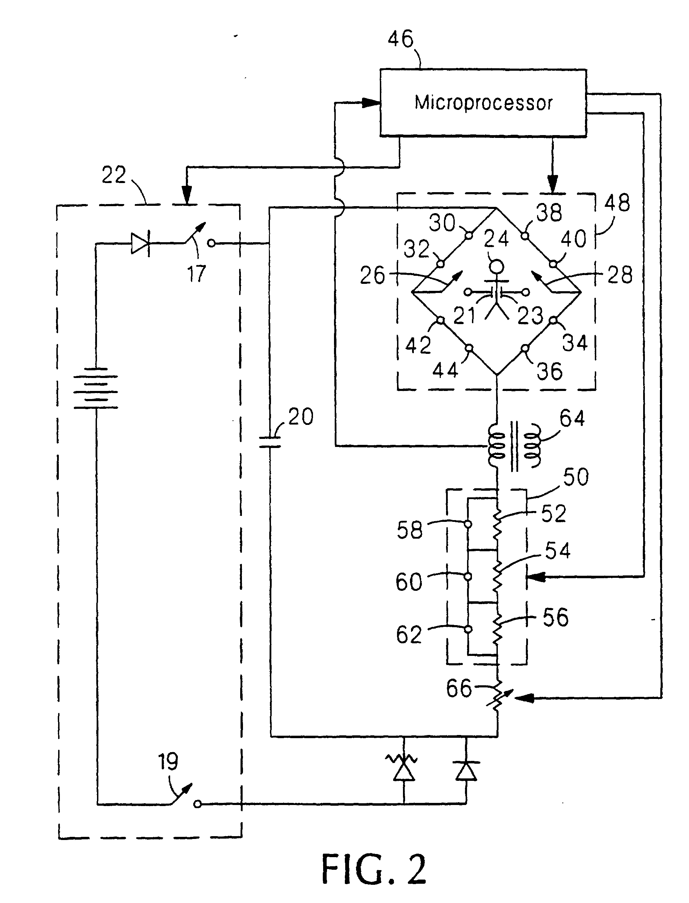

The basic circuitry for producing the biphasic waveform is shown in Fig. 2. A storage capacitor 20 (115 µF) is charged to a maximum of 2200 volts by a charging circuit 22 while relays 26 and 28 and the H-bridge are open, and then the electric charge stored in storage capacitor 20 is allowed to pass through electrodes 21 and 23 and the body of a patient 24. In particular, relay switches 17 and 19 are opened, and then relay switches 26 and 28 are closed. Then, electronic switches 30, 32, 34, and 36 of H-bridge 48 are closed to allow the electric current to pass through the patient's body in one direction, after which electronic H-bridge switches 30, 32, 34, and 36 are opened and H-bridge switches 38, 40, 42, and 44 are closed to allow the electric current to pass through the patient's body in the opposite direction. Electronic switches 30-44 are controlled by signals from respective opto-isolators, which are in turn controlled by signals from a microprocessor 46, or alternatively a hard-wired processor circuit. Relay switches 26, and 28, which are also controlled by microprocessor 46, isolate patient 24 from leakage currents of bridge switches 30-44, which may be about 500 micro-amps. Relay switches 26 and 28 may be relatively inexpensive because they do not have to "hot switch" the current pulse. They close a few milliseconds before H-bridge 48 is "fired" by closure of some of the H-bridge switches.

Electrodes 21 and 23 may be standard defibrillation electrodes having flat surfaces that adhere to the chest of the patient, but they may alternatively be hand-held paddles that are applied to the chest of the patient or hand-held spoons that are applied directly to the patient's heart during open heart surgery. Storage capacitor 20 may be a single capacitor or a set of series-connected or parallel-connected capacitors.

A resistive circuit 50 that includes series-connected resistors 52, 54, and 56 is provided in the current path, each of the resistors being connected in parallel with a shorting switch 58, 60, and 62 controlled by microprocessor 46. The resistors are of unequal value, stepped in a binary sequence to yield 2ⁿ possible resistances where n is the number of resistors. During the initial "sensing pulse," when H-bridge switches 30, 32, 34, and 36 are closed, all of the resistor-shorting switches 58, 60, and 62 are in an open state so that the current passes through all of the resistors in series. Current-sensing transformer 64 senses the current passing through the patient 24, from which microprocessor 46 determines the resistance of the patient 24.

The initial sensing pulse is integral with, i.e., immediately followed by, a biphasic defibrillation waveform, and no re-charging of storage capacitor 20 occurs between the initial sensing pulse and the biphasic defibrillation waveform.

If the patient resistance sensed during the initial sensing pulse is low, all of the resistor-shorting switches 58, 60, and 62 are left open at the end of the sensing pulse so that all of the resistors 52, 54, and 56 remain in the current path (the resistors are then successively shorted out during the positive phase of the biphasic defibrillation waveform in the manner described below in order to approximate a rectilinear positive phase). Thus, the current at the beginning of the positive first phase 12 of the biphasic defibrillation waveform is the same as the current during sensing pulse 10. If the patient resistance sensed during the sensing pulse is high, some or all of the resistor-shorting switches 58, 60, and 62 are closed at the end of the sensing pulse, thereby shorting out some or all of the resistors. This causes an upward jump in current at the end of the sensing pulse as shown in the waveform in Fig. 1.

Thus, immediately after the sensing pulse, the biphasic defibrillation waveform has an initial discharge current that is controlled by microprocessor 46 based on the patient impedance sensed by current-sensing transformer 64.

The current level of the sensing pulse is always at least 50 percent of the current level at the beginning of positive first phase 12, and the sensing pulse, like the defibrillation pulse, is of course a direct-current pulse.

By appropriately selecting the number of resistors that remain in the current path, microprocessor 46 reduces (but does not eliminate) the dependence of peak discharge current on patient impedance, for a given amount of charge stored by the charge storage device.
For a patient resistance of 15 ohms the peak current is about 25 amps, whereas for a patient resistance of 125 ohms the peak current is about 12.5 amps (a typical patient impedance is about 75 ohms).

During the positive phase of the biphasic waveform some or all of the resistors 52, 54, and 56 that remain in series with the patient 24 are successively shorted out. Every time one of the resistors is shorted out, an upward jump in current occurs in the waveform, thereby resulting in the sawtooth ripple shown in the waveform of Fig. 1. The ripple tends to be greatest at the end of the rectilinear phase because the time constant of decay (RC) is shorter at the end of the phase than at the beginning of the phase. Of course, if all of the resistors have already been shorted out immediately after the end of the sensing pulse, the positive phase of the biphasic waveform simply decays exponentially until the waveform switches to the negative phase.

As is shown in Fig. 1, at the end of the positive phase, the current waveform decreases through a series of rapid steps from the end of the positive phase to the beginning of negative phase, one of the steps being at the zero crossing. Microprocessor 46 accomplishes this by 1) successively increasing the resistance of resistive circuit 50 in fixed increments through manipulation of resistor-shorting switches 58, 60, and 62, then 2) opening all of the switches in H-bridge 48 to bring the current waveform down to the zero crossing, then 3) reversing the polarity of the current waveform by closing the H-bridge switches that had previously been open in the positive phase of the current waveform, and then 4) successively decreasing the resistance of resistance circuit 50 in fixed increments through manipulation of resistor-shorting switches 58, 60, and 62 until the resistance of resistance circuit 50 is the same as it was at the end of the positive phase.

In one embodiment a variable resistor 66 is provided in series with the other resistors 52, 54, and 56 to reduce the sawtooth ripple. Every time one of the fixed-value resistors 52, 54, or 56 is shorted out, the resistance of variable resistor 66 automatically jumps to a high value and then decreases until the next fixed-value resistor is shorted out. This tends, to some extent, to smooth out the height of the sawtooth ripple from about 3 amps to about 0.1 to 0.2 amps, and reduces the need for smaller increments of the fixed-value resistors (i.e., it reduces the need for additional fixed-value resistor stages).

The rectilinear phase may exhibit a degree of tilt, either slightly up, or slightly down. This occurs because of the "graininess" of the steps, because patient impedance may change during the waveform, and because of inherent inaccuracies of circuit elements. For example, with respect to graininess of the steps, calculations might show that, for a 50-ohm patient, the optimal resistance required at the end of the rectilinear phase is 14 ohms, in which case we must choose between 10 or 20 ohms based on the available fixed-value resistors. If we choose 10 ohms, an "error" of 4 ohms would result at the end of the rectilinear phase, and the current would rise by about 6 or 7 percent [(14-10)/(50+14)] by the end of the phase. Thus, a 15 amp rectilinear pulse would rise from 15 amps to 16 amps over the rectilinear phase. If it were considered desirable to change this rise to a droop, the microprocessor could easily accommodate such a change. In general, we believe it is desirable to avoid tilt greater than about 20 percent in order to avoid passage of excessive current through the patient's body at the high end of the tilt.

The choices of capacitor (115 µF) and voltage (2200 volts) are based on the desired current requirements and allowable droop during the negative phase. The capacitor stores the minimum energy required to meet the delivered charge requirements (i.e., the charge required to produce the desired current waveform having the desired duration).

The switches in the left-hand side of H-bridge 48 can be tested by closing switches 17 and 19, opening switches 26 and 28, closing switches 30 and 32, then after a short time closing switches 42 and 44, then after a short time opening switches 30 and 32, and then after a short time opening switches 42 and 44. If the switches are working properly, current-sensing transformer 64 will sense the passage of current when all four switches are closed, and will sense no current when switches 30 and 32 or switches 42 and 44 are open. Otherwise, current-sensing transformer 64 will detect the possible presence of a short circuit or an open circuit. Similarly, the switches in the right-hand side of H-bridge 48 can be tested by closing switches 38 and 40, then after a short time closing switches 34 and 36, then after a short time opening switches 38 and 40, and then after a short time opening switches 34 and 36. This valuable safety test does not require current to pass through the patient, due to the placement of current-sensing transformer 64 outside the legs of H-bridge 48.

Microprocessor 46 easily accommodates a complex environment and functions in harmony with various controls, interlocks, and safety features of the electrotherapy system. In addition to performing the functions described herein, the microprocessor may operate a strip chart, a pacer, an ecg monitor, etc. In the event that additional research should show that the characteristics of current pulses should be different from those described herein, the microprocessor can be re-programmed to alter the current waveforms applied to the patient. For example, the microprocessor could accommodate a waveform change to produce a rising or falling rectilinear ramp voltage with time, or a waveform having a negative phase amplitude less than (or greater than) the positive phase amplitude. Of course, the storage capacitor must have enough stored charge to support the required output.

In an alternative embodiment the negative phase of the current waveform is substantially rectilinear, rather than exponentially decaying, and the techniques described above for providing a substantially rectilinear positive phase would be extended to produce the substantially rectilinear negative phase. Such a substantially rectilinear negative phase would require the use of a higher capacitance and voltage and higher-rated switching devices than those employed in the circuit of Fig. 2 (for a given initial current value of the negative phase).

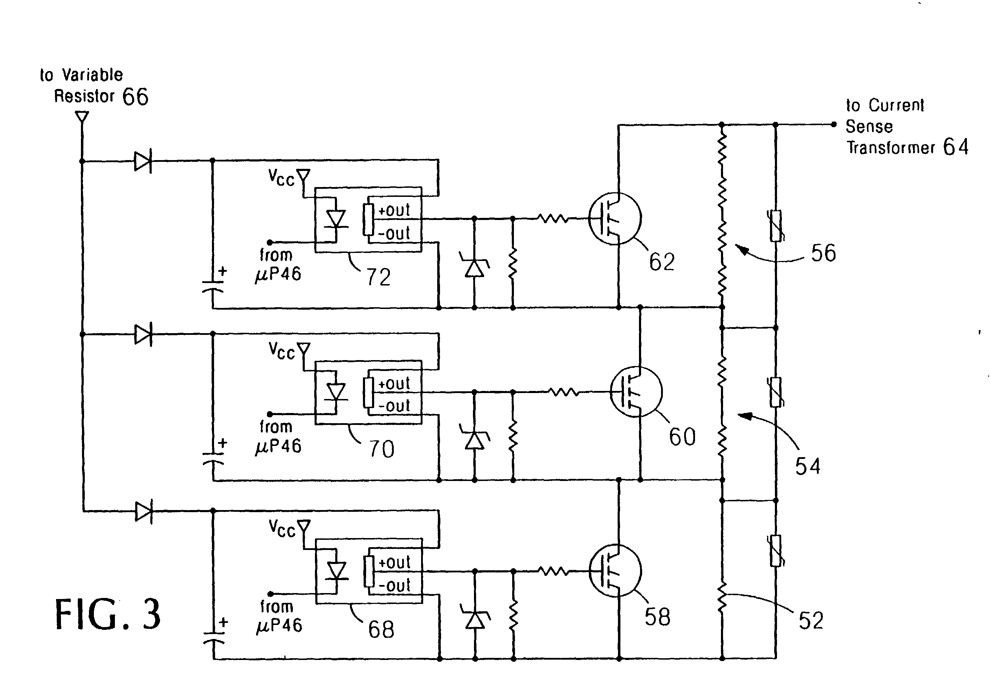

Referring to Fig. 3, the resistive circuit 50 of Fig. 2 includes resistors 52 (10 ohms), 54 (two 10-ohm resistors), and 56 (four 10-ohm resistors) and IGBT shorting switches 58, 60, and 62. Alternatively, other semiconductor switching devices may be used. The resistor string is designed to switch in 10-ohm steps. This allows for a maximum resistance of 80-ohms (including the 10-ohm variable resistor), which makes it possible to limit patient current to 21.5 amps for a 15-ohm patient resistance (the current pulse would be 25.6 amps in the event of a short circuit between the electrodes).

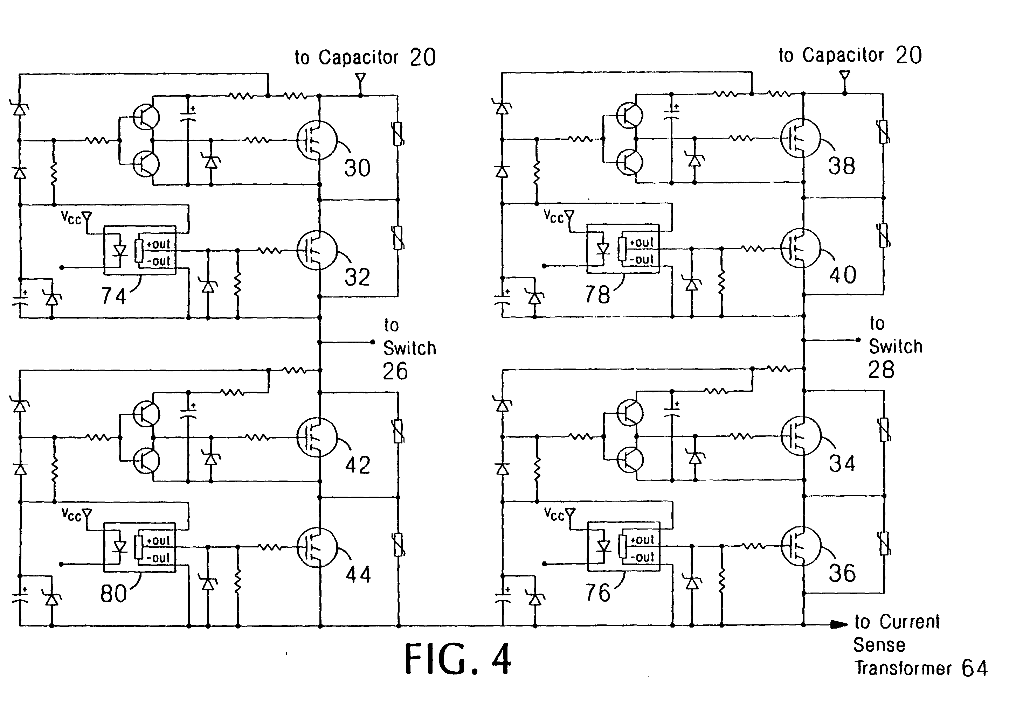

The values of the resistors, as well as the 115 µF value of the storage capacitor and the capacitor voltage of 2200 volts, are determined by the current required to be delivered into the patient load (about 12.5 - 25 amps) and the range of the patient load (e.g., 125 ohms - 15 ohms). The IGBT shorting switches are switched on and off by means of opto-isolator circuits 68, 70, and 72 controlled by the microprocessor. Referring to Fig. 4, H-bridge 48 of Fig. 2 includes IGBT switches 30-44 similarly switched on and off by means of opto-isolator circuits 74, 76, 78, and 80 controlled by the microprocessor. Alternatively, switches 30-44 may be other types of semiconductor switching devices. Only one opto-isolator is provided to control each pair of switches in each arm of the H-bridge.

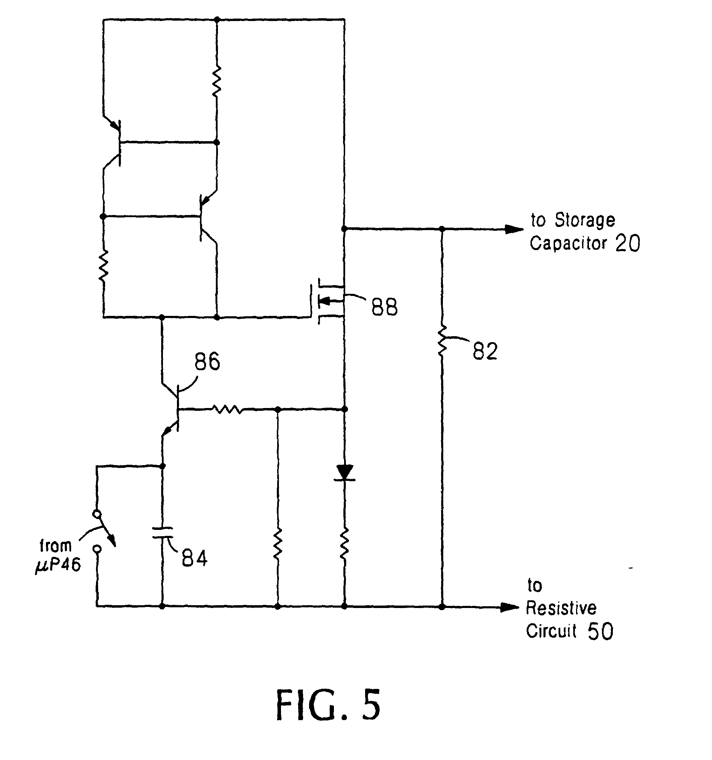

Referring to Fig. 5, variable resistor 66 of Fig. 2 includes resistor 82 connected between resistive circuit 50 and storage capacitor 20. The effective resistance of variable resistor 66 is controlled by the circuitry connected in parallel with resistor 82, through which some of the current from storage capacitor 20 to resistive circuit 50 can pass.

In particular, whenever the microprocessor shorts out one of the fixed-value resistors in resistive circuit 50, it also shorts capacitor 84. This causes transistor 86 to turn on, which pulls the gate of FET or IGBT transistor 88 to ground, thereby turning transistor 88 off. Because transistor 88 is turned off, all of the current from storage capacitor 20 to resistive circuit 50 passes through resistor 82.

Capacitor 84 then begins to charge linearly because of the current source in the collector of transistor 86. This causes the voltage at the drain/collector of transistor 88 to increase linearly, which causes the current in transistor 88 to increase linearly. When the current in transistor 88 increases, the current passing through resistor 82 decreases, thereby reducing the voltage across resistor 82 and therefore reducing the effective resistance of variable resistor 66.

The electrotherapy circuit can be operated in either a "normal" mode of operation or a "high-energy" mode of operation. These two modes of operation are identical when the sensed patient impedance is below 40 ohms. If the sensed patient impedance is above 40 ohms, however, the microprocessor selects an initial resistance value of the series-connected resistors (after the sensing pulse) that depends on the mode of operation. In particular, in the "high-energy" mode of operation the microprocessor selects a lower initial resistance than in the "normal" mode of operation. Thus, more energy will be delivered to the patient in the "high-energy" mode of operation than in the "normal" mode of operation. A practitioner may try to defibrillate in the "normal" mode, then switch to the "high-energy" mode if unsuccessful.

In the "high-energy" mode of operation of the circuit, if the sensed patient impedance is sufficiently high (above 85 ohms) all of the resistor-shorting switches are closed after the initial "sensing pulse," thereby shorting out all of the series-connected resistors. This causes an upward jump at the end of the "sensing pulse," after which the positive and negative phases of the biphasic waveform both decay exponentially.

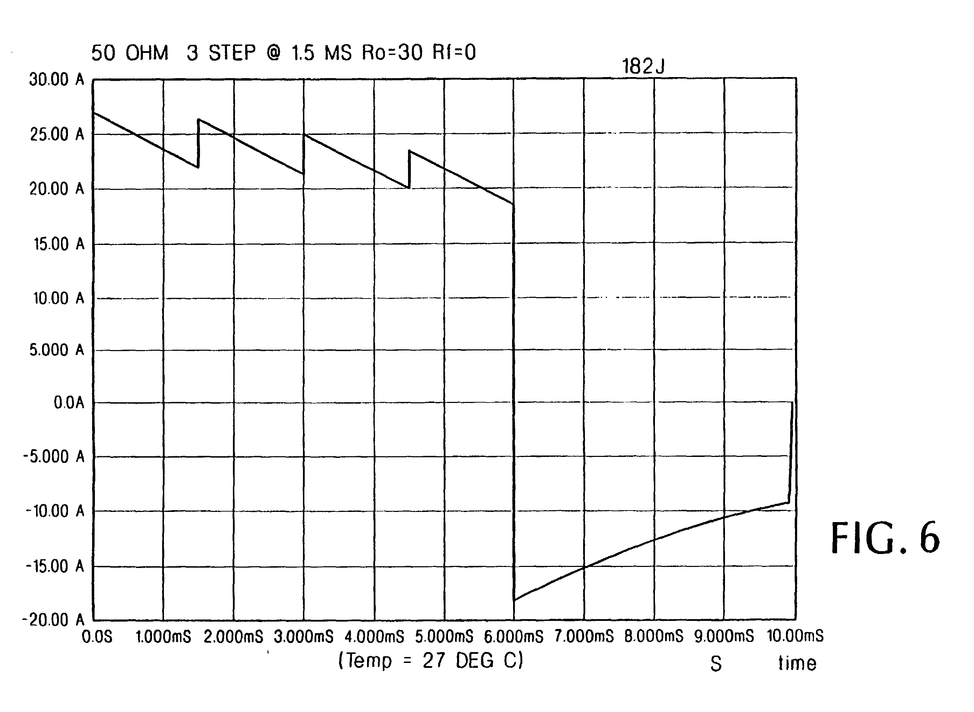

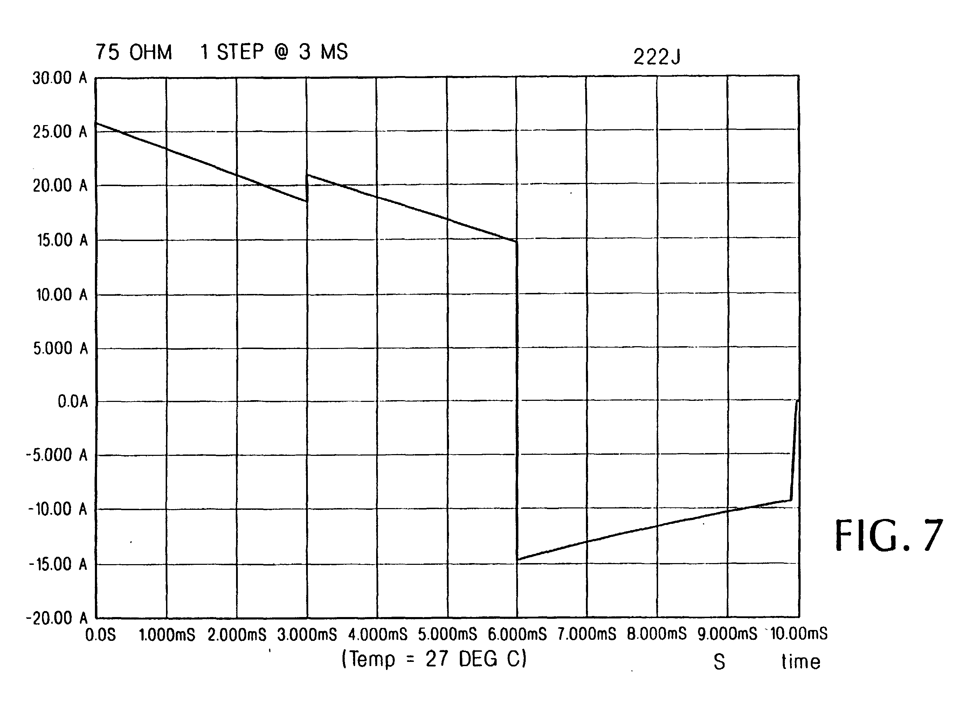

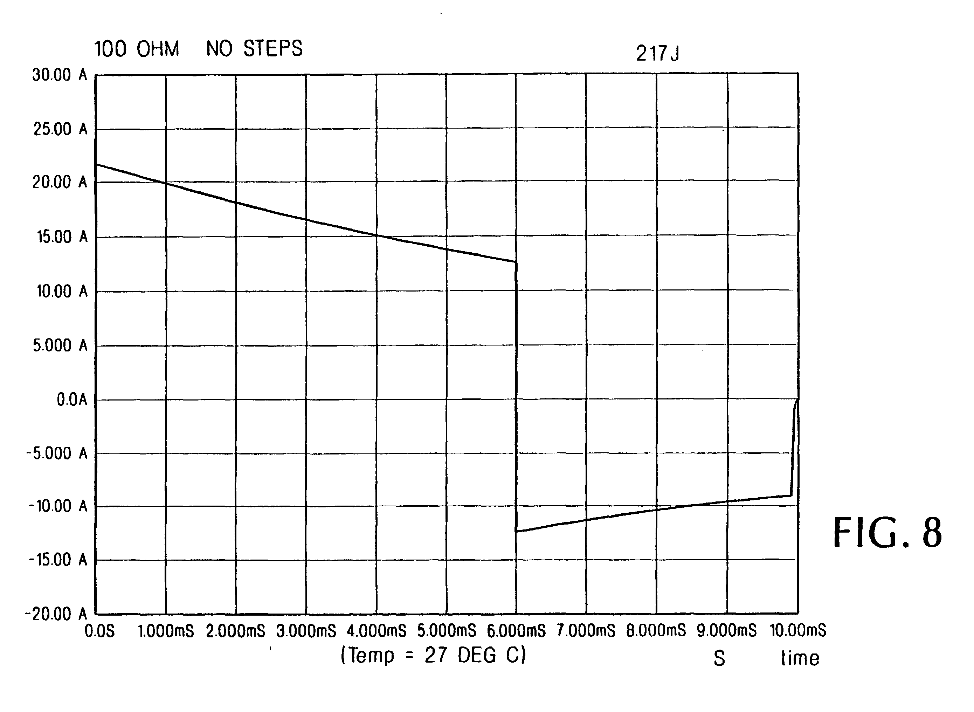

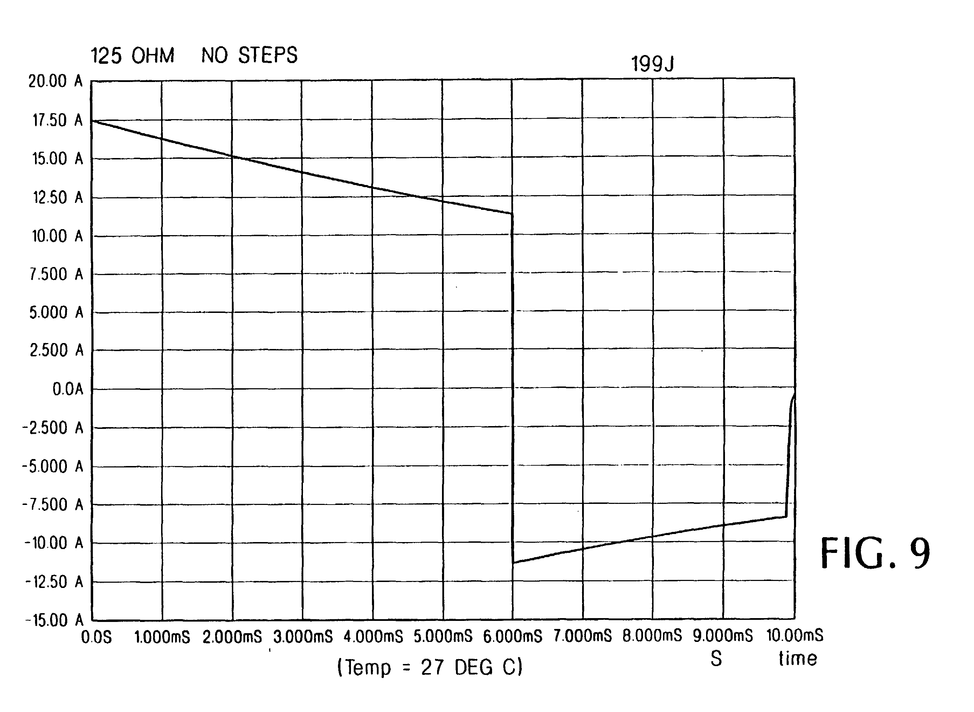

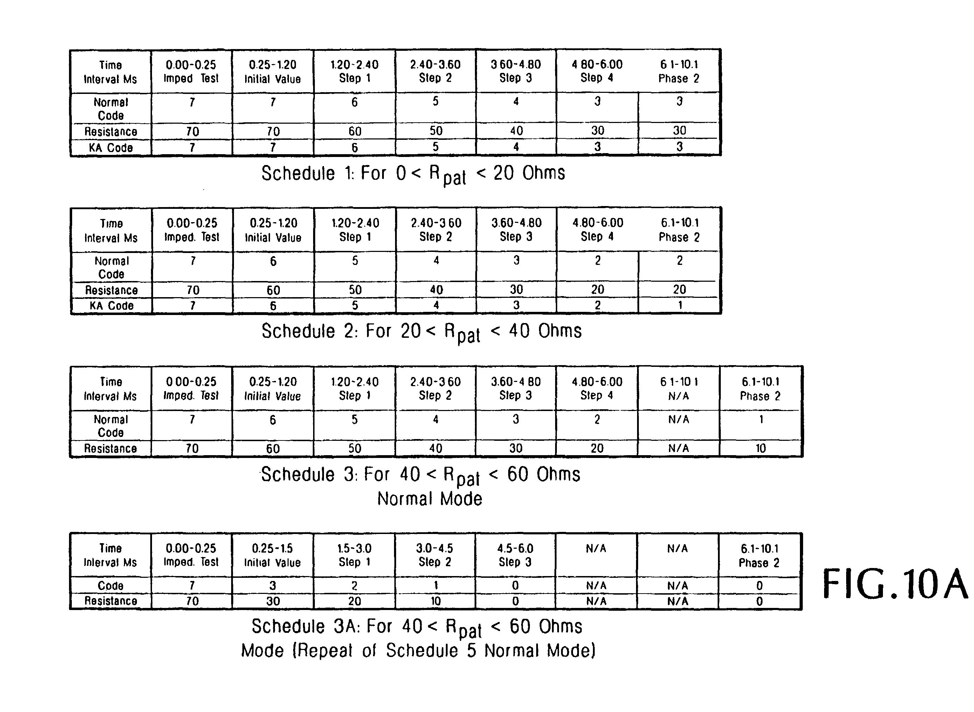

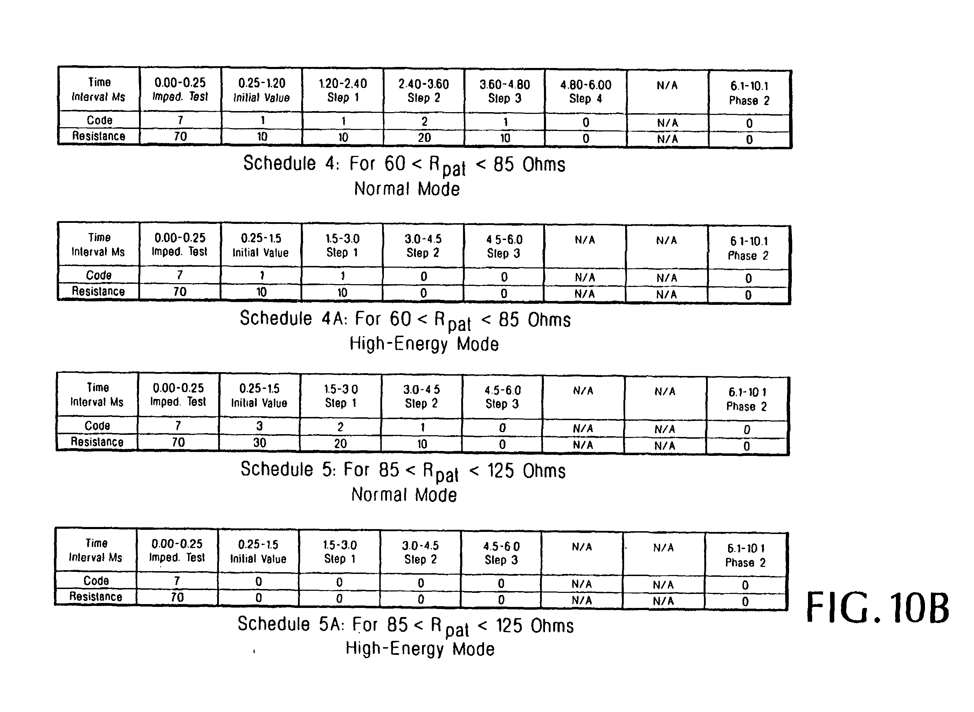

Referring to the table of Figs. 10A and 10B and the waveforms of Figs. 6-9, which correspond to certain schedules in the table of Figs. 10A and 10B, the microprocessor schedules the resistance values of the series-connected resistors based on the measured patient impedance, in a manner such that the stepwise resistance decrease of the series-connected resistors over the course of the rectilinear phase matches the decrease in voltage of the storage capacitor. For the sake of simplicity, the initial sensing pulse and the series of steps between the end of the positive phase and the beginning of negative phase have been omitted from Figs. 6-9, and it is assumed the variable resistor discussed above is not used. Figs. 6-9 are all examples of the "high-energy" mode. Fig. 6, which corresponds to Schedule 3A in Fig. 10A, is based on a patient impedance of 50 ohms, in which case the microprocessor selects an initial series-connected resistance of 30 ohms and a residual series-connected resistance of 0 ohms at the end of the positive phase. The total energy delivered to the patient is about 182 joules. Fig. 7, corresponding to Schedule 4A, is based on a patient impedance of 75 ohms, an initial resistance of 10 ohms, a residual resistance of 0 ohms, and an energy of 222 joules. Fig. 8, corresponding to Schedule 5A, is based on a patient impedance of 100 ohms, an initial resistance of 0 ohms, and a residual resistance of 0 ohms, and an energy of 217 joules. Fig. 9, corresponding to Schedule 5A, is based on a patient impedance of 125 ohms, an initial resistance of 40 ohms, a residual resistance of 0 ohms, and an energy of 199 joules.

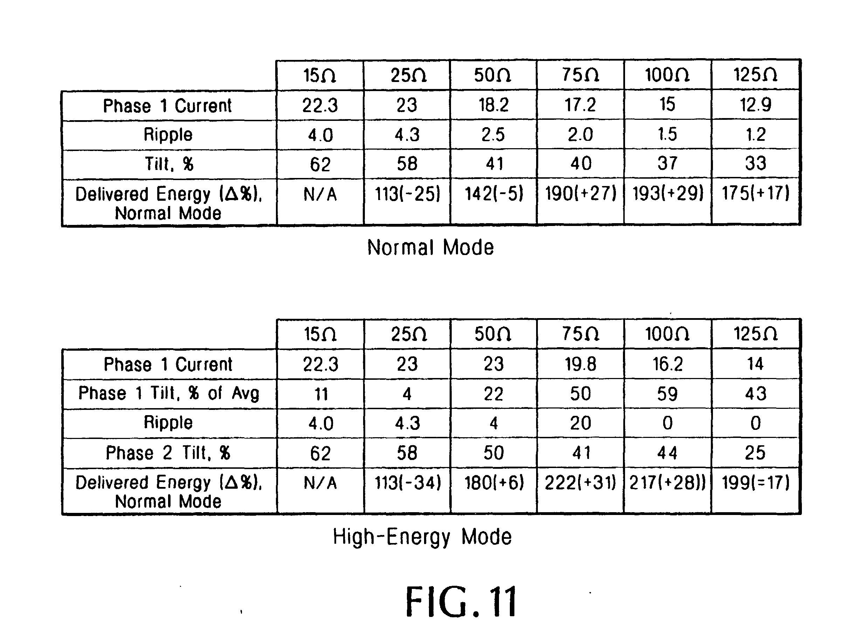

Fig. 11 includes a table, corresponding to the "normal" mode of operation, that identifies, as a function of the patient impedance, the positive-phase current (in amps), ripple (in amps, assuming the variable resistor is not used), tilt of the negative phase (expressed as a percentage of the initial current value of the negative phase), total delivered energy (in joules), and the deviation of the total delivered energy from the normal mode's "rating" of 150 joules. Fig. 11 also includes a similar table for the "high-energy" mode of operation, identifying positive-phase current, tilt of the positive phase (based on a straight-line average through the ripples), ripple, tilt of the negative phase, total delivered energy, and the deviation of the total delivered energy from the "rating" of 170 joules.

In both the high-energy and normal modes described above, the storage capacitor is charged to its maximum voltage of 2200 volts. Other modes of operation can be developed in which the storage capacitor is charged to a lesser voltage, or in which different resistance schedules are used.

The electrotherapy circuits and techniques described herein have a number of applications other than that particularly illustrated. For example, the techniques described herein can be used in connection with implantable defibrillators rather than external defibrillators or in connection with electrotherapy circuits other than defibrillator circuits or even circuits that perform functions other than electrotherapy.

## Claims

1. An electrotherapy circuit for administering to a patient a current waveform, comprising: a charge storage device (20); at least two discharge electrodes (21,23) connected by electrical circuitry to opposite poles of the charge storage device; and a control circuit connected to the charge storage device,
**CHARACTERIZED IN THAT**
the circuit controls a continuous discharge of the charge storage device through the electrodes so as to produce one phase (12) of a current waveform wherein the difference between the peaK current of the phase and the lowest current of the phase is less than one-third of the peak current of the phase, followed by another phase (16) of the current waveform having a polarity opposite to that of the one phase and a duration less than that of the one phase.

2. An electrotherapy circuit according to Claim 1 wherein the other phase of the current waveform having a polarity opposite to that of the one phase, is in the form of a truncated exponential.

3. An electrotherapy circuit according to Claim 1 or Claim 2 wherein the duration of the one phase (12) is between fifty and seventy percent of the combined duration of the one phase (12) and the other phase (16).

4. A circuit according to Claim 3 wherein the duration of the one phase (12) is substantially three-fifths of the combined duration of the one phase (12) and the other phase (16).

5. A circuit according to Claim 3 wherein the duration of the one phase (12) is substantially five-eighths of the combined duration of the one phase (12) and the other phase (16).

6. A circuit according to any preceding Claim wherein the difference between the peak current of the one phase (12) and the lowest current of the phase is less than one-fourth of the peak current of the phase.

7. A circuit according to Claim 6 wherein the difference between the peak current of the one phase (12) and the lowest current of the phase is less than one-fifth of the peak current of the phase.

8. A circuit according to any preceding Claim wherein at least one of said phases (12,16) comprises a ripple (14).

9. A circuit according to Claim 8 wherein the ripple (14) has more than two teeth.

10. A circuit according to Claim 8 or Claim 9 wherein the one phase (12) comprises a ripple (14).

11. A circuit according to any of Claims 8 to 10 wherein the ripple (14) has a height less than one third the height of the peak current of the respective phase (12,16) having the ripple.

12. A circuit according to Claim 11 wherein the ripple (14) has a height less than one fourth the height of the peak current of the respective phase (12,16) having the ripple.

13. A circuit according to Claim 12 wherein the ripple (14) has a height less than one fifth the height of the peak current of the respective phase (12,16) having the ripple.

14. A circuit according to any preceding Claim including a resistive circuit (50) connected between the charge storage device (20) and one of the electrodes (21,23), the control circuit being connected to the resistive circuit (50) to control its resistance during discharge of the charge storage device (20) so as to shape a current waveform produced between the discharge electrodes (21,23).

15. A circuit according to Claim 14 wherein the control circuit controls the resistance of the resistive circuit (50) during discharge of the charge storage device (20) so as to produce a current waveform having at least one rectilinear phase.

16. A circuit according to Claim 15 wherein the rectilinear phase comprises a ripple (14).

17. A circuit according to Claim 15 wherein the rectilinear phase comprises a tilt.

18. A circuit according to Claim 17 wherein the tilt is no more than about twenty percent.

19. A circuit according to any of Claims 14 to 18 wherein the resistive circuit comprises a plurality of discrete resistors (52,54,56).

20. A circuit according to Claim 19 including a switching circuit connected to the plurality of resistors (52,54,56) that selectively provides at least one path for flow of electric current from the charge storage device (20) through a subset of the plurality of resistors to one of the discharge electrodes (21,23).

21. A circuit according to Claim 20 wherein the control circuit controls the switching circuit to select the subset of the resistors through which the electric current flows, the control circuit selecting different subsets of the resistors during different portions of discharge of the charge storage device so as to produce a rectilinear current waveform.

22. A circuit according to Claim 20 or Claim 21 wherein the resistors (52,54,56) are connected in series.

23. A circuit according to Claim 22 wherein the switching circuit selectively provides the path for flow of electric current by shorting out resistors (52,54,56) not in the subset through which the path extends.

24. A circuit according to Claim 23 wherein the resistive circuit (50) includes a variable resistor (66), and the control circuit resets the variable resistor (46) to a high resistance value every time one of the discrete resistors (52,54,56) is shorted out and then allows the variable resistor to decrease to a low resistance value.

25. A circuit according to Claim 23 wherein, at the end of a first phase (12) of the waveform, the switching circuit rapidly and successively adds resistors to the subset through which the path extends to cause the waveform to step down to a value of zero.

26. A circuit according to Claim 25 wherein, at the beginning of the second phase of the waveform, the switching circuit rapidly and successively shorts out resistors in the subset through which the path extends to cause the waveform to step down from the value of zero to a negative value.

27. A circuit according to Claim 25 or Claim 26 wherein the resistors (52,54,56) are stepped in a binary sequence.

28. A circuit according to any of Claims 14 to 27 including at least one switch connected between the charge storage device (20) and one of the electrodes that, when closed, creates a closed circuit for flow of current from the charge storage device to the electrodes.

29. A circuit according to Claim 28 wherein the at least one switch comprises a plurality of switches arranged as an H-bridge (48).

30. A circuit according to any of Claims 14 to 29 wherein the control circuit comprises a microprocessor (46).

31. A circuit according to any of Claims 14 to 30 wherein the control circuit is hard-wired.

32. A circuit according to Claim 14 wherein the resistive circuit (50) comprises a plurality of discrete resistors (52,54,56) and wherein the control circuit selectively provides at least one path for flow of electric current from the charge storage device (20) through a subset of the plurality of resistors to one of the discharge electrodes (21,23).

33. A circuit according to Claim 32 wherein the path for flow of electric current comprises different subsets of the resistors (52,54,56) during different portions of discharge of the charge storage device so as to produce a rectilinear current waveform.

34. A circuit according to Claim 33 wherein the resistors (52,54,56) are connected together in series, the circuit including means (58,60,62) for shorting out resistors not in the subset through which the path extends.

35. A circuit according to any preceding Claim including a variable impedance (66) connected between the charge storage device (20) and one of the electrodes; a sensor (64) that senses a patient-dependent electrical parameter; and a control circuit connected to the sensor and the variable impedance, which circuit controls the impedance of the variable impedance during discharge of the charge storage device based on the patient-dependent electrical parameter sensed by the sensor.

36. A circuit according to Claim 35 wherein the patient-dependent electrical parameter comprises impedance of the patient.

37. A circuit according to Claim 35 or Claim 36 wherein the control circuit controls the variable impedance (66) in a manner so as to reduce dependence of peak discharge current on the patient-dependent electrical parameter.

38. A circuit according to Claim 37 wherein the control circuit selects the impedance of the variable impedance (66) inversely with respect to the impedance of the patient as sensed by the sensor.

39. A circuit according to Claim 38 wherein the peak discharge current if the impedance of the patient is 15 ohms is no more than about twice the peak discharge current if the impedance of the patient is 125 ohms.

40. A circuit according to Claim 37 wherein the discharge of the charge storage device (20) comprises a current waveform having a sensing pulse portion during which the sensor (64) senses the impedance of the patient between the electrodes (21,23), and a therapeutic discharge portion during which the control circuit selects the impedance (66) of the variable impedance inversely with respect to the impedance of the patient as sensed by the sensor (64).

41. A circuit according to Claim 40 wherein the therapeutic discharge portion of the current waveform is rectilinear.

42. A circuit according to any of Claims 35 to 41 wherein the sensor is a current sensor.

43. A circuit according to Claim 42 wherein the sensor (64) is a current sense transformer.

44. A circuit according to any of Claims 35 to 43 wherein the variable impedance (66) comprises a variable resistive circuit.

45. A circuit according to any of Claims 1 to 34 including a sensor (64) for sensing a patient-dependent electrical parameter; and a control circuit, connected to the sensor and the charge storage device (20), which circuit controls discharge of the charge storage device through the electrodes (21,23), based on the patient-dependent electrical parameter as sensed by the sensor, in a manner so as to reduce the dependence of peak discharge current on the electrical parameter for a given amount of charge stored by the charge storage device.

46. A circuit according to Claim 45 including a variable impedance (66) connected between the charge storage device and one of the electrodes (21,23), the control circuit being connected to the variable impedance and controlling discharge of the charge storage device by controlling the variable impedance during discharge of the charge storage device.

47. A circuit according to any of Claims 1 to 34 including a sensor (64) for sensing a patient-dependent electrical parameter; and a control circuit, connected to the sensor and the charge storage device (20), which circuit controls discharge of the charge storage device through the electrodes (21,23), the discharge of the charge storage device comprising a current waveform having a sensing pulse portion, with insufficient energy for performing therapy, during which the sensor (64) senses a patient-dependent electrical parameter, and a therapeutic discharge portion, with sufficient energy for performing therapy, having an initial discharge current controlled by the control circuit based on the patient-dependent electrical parameter as sensed by the sensor, the sensing pulse portion having a discharge current that is at least about one-third of the initial discharge current of the therapeutic discharge portion.

48. A circuit according to Claim 47 wherein the sensing pulse portion has a discharge current that is at least about half the initial discharge current of the therapeutic discharge portion.

49. A circuit according to Claim 47 or Claim 48 wherein the sensing pulse portion has a non-alternating discharge current.

50. A circuit according to any of Claims 47 to 49 wherein the discharge of the charge storage device (20) occurs without recharging of the charge storage device between the sensing pulse portion and the therapeutic discharge portion of the current waveform.

51. A circuit according to any of Claims 47 to 50 wherein the control circuit controls discharge of the charge storage device (20) through the electrodes (21,23), based on the patient-dependent electrical parameter as sensed by the sensor (64), in a manner so as to reduce the dependence of peak discharge current on the patient-dependent electrical parameter during the therapeutic discharge portion of the current waveform.

52. A circuit according to any of Claims 1 to 34 including a sensor (64) for sensing a patient-dependent electrical parameter; and a control circuit, connected to the sensor and the charge storage device (20), which circuit controls discharge of the charge storage device through the electrodes (21,23), the discharge of the charge storage device comprising a current waveform having a sensing pulse portion, with insufficient energy for performing therapy, during which the sensor senses a patient-dependent electrical parameter, and a therapeutic discharge portion, with sufficient energy for performing therapy, having an initial discharge current controlled by the control circuit based on the patient-dependent electrical parameter as sensed by the sensor during the sensing pulse portion, the discharge of the charge storage device occurring without recharging of the charge storage device between the sensing pulse portion and the therapeutic discharge portion of the current waveform.

53. A circuit according to Claim 52 wherein the sensing pulse portion is integral with the therapeutic discharge portion.

54. A circuit according to any of Claims 1 to 34 including a sensor (64) for sensing a patient-dependent electrical parameter during a sensing pulse portion of a current waveform, the sensing pulse portion having insufficient energy for performing therapy; and a control circuit, connected to the sensor (64) and the charge storage device (20) for controlling discharge of the charge storage device through the electrodes (21,23), the discharge of the charge storage device comprising the current waveform having the sensing pulse portion during which the sensor senses a patient-dependent electrical parameter, the current waveform also having a therapeutic discharge portion, with sufficient energy for performing therapy, having an initial discharge current controlled by the control circuit based on the patient-dependent electrical parameter as sensed by the sensor, the sensing pulse portion of the current waveform being integral with the therapeutic discharge portion of the current waveform.

55. A circuit according to any preceding Claim wherein the electrodes (21,23) are non-implanted electrodes.

56. A circuit according to any preceding Claim wherein the electrodes are transthoracic electrodes.

57. A circuit according to any preceding Claim wherein the electrodes (21,23) are adapted for application externally to a patient.

58. A circuit according to Claim 57 wherein the electrodes (21,23) are adapted to be applied to a patient's heart during surgery.

59. A circuit according to any preceding Claim adapted to apply the current as a defibrillation waveform.

60. A circuit according to any preceding Claim wherein the charge storage device (20) comprises at least one capacitor.

61. A circuit according to Claim 60 wherein the charge storage device (20 is a single capacitor.

## Patentansprüche

1. Elektrotherapiestramkreis zur Verabreichung eines Stroms in Wellenform an einen Patienten, der Folgendes umfasst: eine Ladungsspeichervorrichtung (20); mindestens zwei Entladungselektroden (21, 23), die durch eine elektrische Schaltungsanordnung mit entgegengesetzten Polen mit der Ladungsspeichervorrichtung verbunden sind; und einen Steuerstromkreis.
**DADURCH GEKENNZEICHNET, DASS**
der Stromkreis eine kontinuierliche Entladung der Ladungsspeichervorrichtung so über die Elektroden steuert, dass eine erste Phase (12) eines Stroms in Wellenform erzeugt wird, wobei die Differenz zwischen dem Spitzenstrom der Phase und dem geringsten Strom der Phase weniger als ein Drittel des Spitzenstroms der Phase beträgt, gefolgt von einer zweiten Phase (16) des Stroms in Wellenform, deren Polung derjenigen der ersten Phase entgegengesetzt ist und die eine kürzere Dauer als die erste Phase hat.

2. Elektrotherapiestromkreis nach Anspruch 1, wobel die zweite Phase des Stroms in Wellenform, deren Polung derjenigen der ersten Phase entgegengesetzt ist, die Form einer abgekürzten Exponentialfunktion hat.

3. Elektrotherapiestromkreis nach Anspruch 1 oder Anspruch 2. wobei die Dauer der ersten Phase (12) zwischen fünfzig und siebzig Prozent der Gesamtdauer der ersten Phase (12) und der zweiten Phase (16) beträgt.

4. Stromkreis nach Anspruch 3, wobel die Dauer der ersten Phase (12) im Wesentlichen drei Fünftel der Gesamtdauer der ersten Phase (12) und der zweiten Phase (16) beträgt.

5. Stromkreis nach Anspruch 3, wobei die Dauer der ersten Phase (12) im Wesentlichen fünf Achtel der Gesamtdauer der ersten Phase (12) und der zweiten Phase (16) beträgt.

6. Stromkreis nach jedem vorherigen Anspruch, wobei die Differenz zwischen dem Spitzenstrom der ersten Phase (12) und dem geringsten Strom der Phase weniger als ein Viertel des Spitzenstroms der Phase beträgt.

7. Stromkreis nach Anspruch 6, wobei die Differenz zwischen dem Spitzenstrom der ersten Phase (12) und dem geringsten Strom der Phase weniger als ein Fünftel des Spitzenstroms der Phase beträgt.

8. Stromkreis nach jedem vorherigen Anspruch, wobei mindestens eine der Phasen (12, 16) eine Welligkeit (14) umfasst.

9. Stromkreis nach Anspruch 8, wobei die Welligkeit (14) mehr als zwei Spitzen hat.

10. Stromkreis nach Anspruch 8 oder Anspruch 9. wobei die erste Phase (12) eine Welligkeit (14) umfasst.

11. Stromkreis nach jedem der Ansprüche B bis 10, wobei die Höhe der Welligkeit (14) geringer als ein Drittel des Spitzenstroms der eine Welligkeit aufweisenden jeweiligen Phase (12, 16) ist.

12. Stromkreis nach Anspruch 11, wobei die Höhe der Welligkeit (14) geringer als ein Viertel des Spitzenstroms der eine Welligkeit aufweisenden jeweiligen Phase ist.

13. Stromkreis nach Anspruch 12, wobei die Höhe der Welligkeit (14) geringer als ein Fünftel des Spitzenstroms der eine Welligkeit aufweisenden jeweiligen Phase (12, 16) ist.

14. Stromkreis nach jedem vorherigen Anspruch einschließlich eines mit der Ladungsspeichervorrichtung (20) und einer der Elektroden (21. 23) verbundenen ohmschen Stromkreises (50), wobei der Steuerstromkreis mit dem ohmschen Stromkreis (50) verbunden ist, damit sein Widerstand während der Entladung der Ladungsspeichervorrichtung (20) geregelt werden kann, so dass zwischen den Entladungselektroden (21, 23) ein Strom mit einer bestimmten Wellenform erzeugt wird.

15. Stromkreis nach Anspruch 14, wobei der Steuerstromkreis den Widerstand des ohmschen Stromkreises (50) während der Entladung der Ladungsspeichervorrichtung (20) so regelt, dass ein Strom in Wellenform mit mindestens einer linearen Phase erzeugt wird.

16. Stromkreis nach Anspruch 15, wobei die lineare Phase eine Welligkeit (14) umfasst.

17. Stromkreis nach Anspruch 15, wobei die lineare Phase eine Neigung umfasst.

18. Stromkreis nach Anspruch 17, wobei die Neigung nicht mehr als etwa zwanzig Prozent beträgt.

19. Stromkreis nach jedem der Ansprüche 14 bis 18, wobei der ohmsche Stromkreis eine Vielzahl diskreter Widerstände (52, 54, 56) umfasst.

20. Stromkreis nach Anspruch 19, wobei er einen mit mehreren Widerständen (52, 54, 56) verbundenen Schaltkreis einschließt, der mindestens einen Pfad zur Verfügung stellt, auf dem der elektrische Strom von der Ladungsspelchervorrichtung (20) über eine Teilmenge der Widerstände zu einer der Entladungselektroden (21, 23) fließt.

21. Stromkreis nach Anspruch 20, wobei der Steuerstromkreis den Schaltkreis so regelt, dass er die Teilmenge der Widerstände, durch die der elektrische Strom fließt, auswählt. Der Steuerstromkreis steuert dabei verschiedene Teilmengen der Widerstände während der verschiedenen Abschnitte der Entladung der Ladungsspeichervorrichtung so, dass Strom in linearer Wellenform erzeugt wird.

22. Stromkreis nach Anspruch 20 oder 21, wobei die Widerstände (52, 54, 56) in Reihe geschaltet sind.

23. Stromkreis nach Anspruch 22, wobei der Schaltkreis den Pfad für das Fließen des elektrischen Stroms selektiv zur Verfügung stellt, Indem er die für den Strompfad nicht erforderlichen Widerstände (52, 54, 56) überbrückt.

24. Stromkreis nach Anspruch 23, wobei der ohmsche Stromkreis (50) einen Stellwiderstand (66) umfasst und der Steuerstromkreis (46) jedes Mal, wenn einer der diskreten Widerstände (52, 54, 56) überbrückt wird, den Stellwiderstand auf einen hohen Widerstandswert zurückstellt und ihm danach gestattet, auf einen geringen Widerstandswert zu sinken.

25. Stromkreis nach Anspruch 23, wobei der Schaltkreis am Ende einer ersten Phase (12) der Wellenform schnell und nacheinander Widerstände zu der Teilmenge hinzufügt, durch die der Pfad verläuft. so dass die Wellenform schrittweise auf einen Wert von Null reduziert wird.

26. Stromkreis nach Anspruch 25, wobei der Schaltkreis zu Beginn der zweiten Phase der Wellenform schnell und nacheinander Widerstände in der Teilmenge, durch die der Pfad verläuft, überbrückt, so dass die Wellenform vom Nullwert auf einen negativen Wert reduziert wird.

27. Stromkreis nach Anspruch 25 oder Anspruch 26, wobei die Widerstände (52, 54, 56) in binärer Folge gestaffelt sind.

28. Stromkreis nach jedem der Ansprüche 14 bis 27, der mindestens einen Schalter zwischen der Ladungsspeichervorrichtung (20) und einer der Elektroden umfasst, der in geschlossenem Zustand einen geschlossenen Stromkreis ergibt, in dem der Strom von der Ladungsspeichervorrichtung zu den Elektroden fließt.

29. Stromkreis nach Anspruch 28, wobei mindestens ein Schalter eine Vielzahl von Schaltern umfasst, die als H-Brücke (48) angeordnet sind.

30. Stromkreis nach jedem der Ansprüche 14 bis 29, wobei der Steuerstromkreis einen Mikroprozessor (46) umfasst.

31. Stromkreis nach jedem der Ansprüche 14 bis 30, wobei der Steuerstromkreis fest verdrahtet ist.

32. Stromkreis nach Anspruch 14, wobei der ohmsche Stromkreis (50) mehrere diskrete Widerstände (52, 54, 56) umfasst und der Steuerstromkreis seiektiv mindestens einen Pfad zur Verfügung stellt, auf dem der elektrische Strom von der Ladungsspeichervorrichtung (20) durch eine Teilmenge der Widerstände zu einer der Entladungselektroden (21, 23) fließt.

33. Stromkreis nach Anspruch 32, wobei der elektrische Strompfad während verschiedener Abschnitte der Entladung der Ladungsspeichervorrichtung verschiedene Teilmengen von Widerständen (52, 54, 56) umfasst, so dass Strom in linearer Wellenform erzeugt wird

34. Stromkreis nach Anspruch 33, wobei die Widerstände (52. 54, 56) in Reihe zusammengeschaltet sind und der Stromkreis Überbrückungen (58, 60, 62) von Widerständen umfasst, die nicht zu der Teilmenge gehören, durch die der Pfad verläuft.

35. Stromkreis nach jedem vorherigen Anspruch, bestehend aus einem mit der Ladungsspeichervorrichtung (20) und einer der Elektroden verbundenen regelbaren Wechselstromwiderstand (66), einem Messwertgeber (64), der einen patientenabhängigen elektrischen Parameter abtastet, und einem mit dem Messwertgeber und dem regelbaren Wechselstromwiderstand verbundenen Steuerstromkreis, der den Scheinwiderstand des regelbaren Wechselstromwiderstandes während der Entladung der Ladungsspeichervorrichtung aufgrund der patientenabhängigen, vom Messwertgeber abgetasteten elektrischen Parameter steuert.

36. Stromkreis nach Anspruch 35, wobei der patientenabhängige elektrische Parameter den Scheinwiderstand des Patienten umfasst.

37. Stromkreis nach Anspruch 35 oder Anspruch 36. wobei der Steuerstromkreis den regelbaren Wechselstromwiderstand (66) steuert, um die Abhängigkeit des Entladungsspitzenstroms von dem patientenabhängigen Parameter zu verringern.

38. Stromkreis nach Anspruch 37, wobei der Steuerstromkreis den Scheinwiderstand des regelbaren Wechselstromwiderstandes (66) umgekehrt zu dem vom Messwertgeber abgetasteten Scheinwiderstand des Patienten auswählt.

39. Stromkreis nach Anspruch 38, wobei im Falle eines Scheinwiderstands des Patienten von 15 Ohm der Entladungsspitzenstrom nicht mehr als etwa das Doppelte des Entladungsspitzenstroms bei einem Scheinwiderstand des Patienten von 125 Ohm beträgt.

40. Stromkreis nach Anspruch 37, wobei die Entladung der Ladungsspeichervorrichtung (20) einen Strom in Wellenform mit einem Abtastimpulsabschnitt, in dessen Verlauf der Messwertgeber (64) den Scheinwiderstand des Patienten zwischen den Elektroden (21, 23) abtastet, und einen therapeutischen Entladungsabschnitt umfasst, in dessen Verlauf der Steuerstromkreis den Scheinwiderstand (66) des regelbaren Wechselstromwiderstands umgekehrt zu dem vom Messwertgeber (64) abgetasteten Scheinwiderstand des Patienten auswählt.

41. Stromkreis nach Anspruch 40, wobei der therapeutische Entladungsabschnitt des Stroms in Wellenform linear ist.

42. Stromkreis nach jedem der Ansprüche 35 bis 41, wobei der Messwertgeber ein Stromgeber ist.

43. Stromkreis nach Anspruch 42, wobei der Messwertgeber (64) ein Stromrichtungsumformer ist.

44. Stromkreis nach jedem der Ansprüche 35 bis 43, wobei der regelbare Wechsetstromwiderstand (66) einen regelbaren ohmschen Stromkreis umfasst.

45. Stromkreis nach jedem der Ansprüche 1 bis 34 einschließlich eines Messwertgebers (64) zum Abtasten eines patientenabhangigen elektrischen Parameters und eines mit dem Messwertgeber und der Ladungsspeichervorrichtung (20) verbundenen Steuerstromkreises, der die Entladung der Ladungsspeichervorrichtung über die Elektroden (21. 23) aufgrund des vom Messwertgeber ermittelten patientenabhänglgen elektrischen Parameters steuert, so dass die Abhängigkeit des Entladungsspitzenstroms von dem elektrischen Parameter für einen festgelegten Teil der von der Ladungsspeichervorrichtung gespeicherten Ladung verringert wird.

46. Stromkreis nach Anspruch 45 einschließlich eines regelbaren Wechselstromwiderstands (66) zwischen der Ladungsspeichervorrichtung und einer der Elektroden (21, 23), wobei der Steuerstromkreis mit dem regelbaren Wechselstromwiderstand verbunden ist und die Entladung der Ladungsspeichervorrichtung durch Regelung des regelbaren Wechselstromwiderstands während der Entladung der Ladungsspeichervorrichtung steuert.

47. Stromkreis nach jedem der Ansprüche 1 bis 34 einschließlich eines Messwertgebers (64) zum Abtasten eines patientenabhängigen elektrischen Parameters und eines mit dem Messwertgeber und der Ladungsspeichervorrichtung (20) verbundenen Steuerstromkreises. der die Entladung der Ladungsspeichervorrichtung über die Elektroden (21, 23) steuert, die Entladung der Ladungsspeichervorrichtung dabei einen Strom in Wellenform mit einem Abtastimpulsabschnitt, dessen Energie während des Abtastens eines patientenabhängigen elektrischen Parameters durch den Messwertgeber (64) nicht für die Therapie ausreicht, und einem therapeutischen Entladungsabschnitt mit ausreichender Energie für die Durchführung der Therapie umfasst, wobei ein Anfangsentladungsstrom aufgrund des vom Messwertgeber abgetasteten patientenabhängigen elektrischen Parameters vom Steuerstromkreis gesteuert wird und der Abtastimpulsabschnitt einen Entladungsstrom hat, der mindestens etwa ein Drittel des Anfangsentladungsstroms des therapeutischen Entladungsabschnitts beträgt.

48. Stromkreis nach Anspruch 47, wobei der Entladungsstrom des Abtastimpulsabschnitts mindestens etwa die Hälfte des Anfangsentladungsstroms des therapeutischen Entladungsabschnitts beträgt,

49. Stromkreis nach Anspruch 47 oder Anspruch 48, wobei der Abtastimpulsabschnitt einen nicht wechselnden Entladungsstrom hat.

50. Stromkreis nach jedem der Ansprüche 47 bis 49, wobei die Entladung der Ladungsspeichervorrichtung (20) eintritt, ohne dass zwischen dem Abtastimpulsabschnitt und dem therapeutischen Entladungsabschnitt des Stroms in Wellenform eine Aufladung der Ladungsspeichervorrichtung (20) erfolgt.

51. Stromkreis nach jedem der Ansprüche 47 bis 50, wobei der Steuerstromkreis die Entladung der Ladungsspeichervorrichtung (20) über die Elektroden (21, 23) aufgrund des vom Messwertgeber (64) abgetasteten patientenabhängigen elektrischen Parameters steuert, so dass die Abhängigkeit des Entladungsspitzenstroms von dem patientenabhängigen elektrischen Parameter während des therapeutischen Entladungsabschnitts des Stroms In Wellenform verringert wird.

52. Stromkreis nach jedem der Ansprüche 1 bis 34 einschließlich eines Messwertgebers (64) zum Abtasten eines patientenabhängigen elektrischen Parameters und eines mit dem Messwertgeber und der Ladungsspeichervorrichtung (20) verbundenen Steuerstromkreises, der die Entladung der Ladungsspeichervorrichtung über die Elektroden (21, 23) steuert, die Entladung der Ladungsspeichervorrichtung dabei einen Strom in Wellenform mit einem Abtastimpulsabschnitt, dessen Energie während des Abtastens eines patientenabhängigen elektrischen Parameters durch den Messwertgeber nicht für die Therapie ausreicht, und einem therapeutischen Entladungsabschnitt mit ausreichender Energie für die Durchführung der Therapie umfasst, wobei ein Anfangsentladungsstrom aufgrund des vom Messwertgeber während des Abtastimpulsabschnitts abgetasteten patientenabhängigen elektrischen Parameters vom Steuerstromkreis gesteuert wird und die Entladung der Ladungsspeichervorrichtung erfolgt, ohne dass die Ladungsspeichervorrichtung zwischen dem Abtastimpulsabschnitt und dem therapeutischen Entladungsimpulsabschnitt des Stroms in Wellenform aufgeladen wird.

53. Stromkreis nach Anspruch 52, wobei der Abtastimpulsabschnitt Bestandteil des therapeutischen Entladungsabschnitts ist.

54. Stromkreis nach jedem der Ansprüche 1 bis 34 einschließlich eines Messwertgebers (64) zum Abtasten eines patientenabhängigen elektrischen Parameters während eines Abtastimpulsabschnitts eines Stroms in Wellenform, in dem die Energie nicht für die Durchführung der Therapie ausreicht, und eines mit dem Messwertgeber (84) und der Ladungsspeichervorrichtung (20) verbundenen Steuerstromkreises zur Steuerung der Entladung der Ladungsspeichervorrichtung über die Elektroden (21. 23), wobei die Entladung der Ladungsspeichervorrichtung einen Strom in Wellenform mit einem Abtastimputsabschnitt, in dessen Verlauf der Messwertgeber einen patlentenabhängigen elektrischen Parameter abtastet, und ferner mit einem therapeutischen Entladungsabschnitt mit ausreichender Energie für die Durchführung der Therapie umfasst, ein Anfangsentladungsstrom aufgrund des vom Messwertgeber abgetasteten patientenabhängigen elektrischen Parameters vom Steuerstromkreis gesteuert wird und der Abtastimpulsabschnitt des Stroms in Wellenform Bestandteil des therapeutischen Entladungsabschnitts des Stroms in Wellenform ist.

55. Stromkreis nach jedem vorherigen Anspruch, wobei die Elektroden (21, 23) nicht implantiart sind.

56. Stromkreis nach jedem vorherigen Anspruch, wobei die Elektroden transthorakale Elektroden sind.

57. Stromkreis nach jedem vorherigen Anspruch, wobei die Elektroden (21, 23) für die äußere Anwendung bei einem Patienten geeignet sind.

58. Stromkreis nach Anspruch 57, wobei die Elektroden (21, 23) für die Anwendung am Herzen eines Patienten während eines chirurgischen Eingriffs geeignet sind.

59. Stromkreis nach jedem vorherigen Anspruch, der für den Einsatz des Stroms als Defibrillationswellenform geeignet ist.

60. Stromkreis nach jedem vorherigen Anspruch, wobei die Ladungsspeichervorrichtung (20) mindestens einen Kondensator umfasst.

61. Stromkreis nach Anspruch 60, wobei die Ladungsspeichervorrichtung (20) ein einzelner Kondensator ist.

## Revendications

1. Circuit d'électrothérapie pour administrer une forme d'onde de courant à un patient, comprenant : un dispositif à stockage de charge (20) ; au moins deux électrodes de décharge (21, 23) connectées par des circuits électriques à des pôles opposés du dispositif à stockage de charge ; et un circuit de commande connecté au dispositif à stockage de charge,
**caractérisé en ce que**
le circuit commande une décharge continue du dispositif à stockage de charge au travers des électrodes afin de produire une phase (12) d'une forme d'onde de courant, dans laquelle la différence entre le courant maximal de la phase et le courant minimal de la phase est inférieure à un tiers du courant maximal de la phase, suivie d'une autre phase (16) de la forme d'onde de courant présentant une polarité opposée à celle de la phase (12) et une durée inférieure à celle de cette phase.

2. Circuit d'électrothérapie selon la revendication 1, dans lequel l'autre phase de la forme d'onde de courant présentant une polarité opposée à celle de la phase a une forme exponentielle tronquée.

3. Circuit d'électrothérapie selon la revendication 1 ou la revendication 2, dans lequel la durée de la phase (12) est comprise entre cinquante et soixante-dix pour cent de la durée combinée de la phase (12) et de l'autre phase (16).

4. Circuit selon la revendication 3, dans lequel la durée de la phase (12) représente pour l'essentiel trois cinquièmes de la durée combinée de la phase (12) et de l'autre phase (16).

5. Circuit selon la revendication 3, dans lequel la durée de la phase (12) représente pour l'essentiel cinq huitièmes de la durée combinée de la phase (12) et de l'autre phase (16).

6. Circuit selon l'une quelconque des revendications précédentes, dans lequel la différence entre le courant maximal de la phase (12) et le courant minimal de la phase est inférieure à un quart du courant maximal de la phase.

7. Circuit selon la revendication 6, dans lequel la différence entre le courant maximal de la phase (12) et le courant minimal de la phase est inférieure à un cinquième du courant maximal de la phase.

8. Circuit selon l'une quelconque des revendications précédentes, dans lequel au moins l'une desdites phases (12, 16) comprend une ondulation (14).

9. Circuit selon la revendication 8, dans lequel l'ondulation (14) a plus de deux dents.

10. Circuit selon la revendication 8 ou la revendication 9, dans lequel la phase (12) comprend une ondulation (14).

11. Circuit selon l'une quelconque des revendications 8 à 10, dans lequel l'ondulation (14) a une hauteur inférieure à un tiers de la hauteur du courant maximal de la phase respective (12, 16) présentant l'ondulation.

12. Circuit selon la revendication 11, dans lequel l'ondulation (14) a une hauteur inférieure à un quart de la hauteur du courant maximal de la phase respective (12, 16) présentant l'ondulation.

13. Circuit selon la revendication 12, dans lequel l'ondulation (14) a une hauteur inférieure à un cinquième de la hauteur du courant maximal de la phase respective (12, 16) présentant l'ondulation.

14. Circuit selon l'une quelconque des revendications précédentes comprenant un circuit à résistance (50) connecté entre le dispositif à stockage de charge (20) et l'une des électrodes (21, 23), le circuit de commande étant connecté au circuit à résistance (50) pour commander sa résistance pendant la décharge du dispositif à stockage de charge (20) afin de former une forme d'onde de courant produite entre les électrodes de décharge (21, 23).

15. Circuit selon la revendication 14, dans lequel le circuit de commande commande la résistance du circuit à résistance (50) pendant la décharge du dispositif à stockage de charge (20) afin de produire une forme d'onde de courant ayant au moins une phase rectiligne.

16. Circuit selon la revendication 15, dans lequel la phase rectiligne comprend une ondulation (14).

17. Circuit selon la revendication 15, dans lequel la phase rectiligne comprend une inclinaison.

18. Circuit selon la revendication 17, dans lequel l'inclinaison n'est pas supérieure à vingt pour cent environ.

19. Circuit selon l'une quelconque des revendications 14 à 18, dans lequel le circuit à résistance comprend une pluralité de résistances distinctes (52, 54, 56).

20. Circuit selon la revendication 19 comprenant un circuit de mise en marche et d'arrêt connecté à la pluralité de résistances (52, 54, 56) qui fournit sélectivement au moins un cheminement pour l'écoulement du courant électrique, en partant du dispositif à stockage de charge (20) et en passant par un sous-ensemble de la pluralité de résistances, jusqu'à l'une des électrodes de décharge (21, 23).

21. Circuit selon la revendication 20, dans lequel le circuit de commande commande le circuit de mise en marche et d'arrêt pour sélectionner le sous-ensemble de résistances qui sera traversé par le courant électrique, le circuit de commande sélectionnant différents sous-ensembles de résistances pendant différentes parties de décharge du dispositif à stockage de charge afin de produire une forme d'onde de courant rectiligne.

22. Circuit selon la revendication 20 ou la revendication 21, dans lequel les résistances (52, 54, 56) sont connectées en série.

23. Circuit selon la revendication 22, dans lequel le circuit de mise en marche et d'arrêt fournit sélectivement le cheminement pour l'écoulement du courant électrique en court-circuitant des résistances (52, 54, 56) ne se trouvant pas dans le sous-ensemble traversé par le cheminement.

24. Circuit selon la revendication 23, dans lequel le circuit à résistance (50) comprend une résistance variable (66), et le circuit de commande réarme la résistance variable (46) sur une valeur de résistance élevée à chaque fois que l'une des résistances distinctes (52, 54, 56) est court-circuitée et permet ainsi de diminuer la résistance variable sur une valeur de faible résistance.

25. Circuit selon la revendication 23, dans lequel, à la fin d'une première phase (12) de la forme d'onde, le circuit de mise en marche et d'arrêt ajoute des résistances rapidement et successivement au sous-ensemble traversé par le cheminement pour que la forme d'onde s'abaisse sur une valeur de zéro.

26. Circuit selon la revendication 25, dans lequel, au début de la seconde phase de la forme d'onde, le circuit de mise en marche et d'arrêt court-circuite des résistances rapidement et successivement dans le sous-ensemble traversé par le cheminement pour que la forme d'onde s'abaisse de la valeur de zéro sur une valeur négative.

27. Circuit selon la revendication 25 ou la revendication 26, dans lequel les résistances (52, 54, 56) sont abaissées dans une séquence binaire.

28. Circuit selon l'une quelconque des revendications 14 à 27 comprenant au moins un commutateur connecté entre le dispositif à stockage de charge (20) et l'une des électrodes qui, lorsqu'il est fermé, crée un circuit fermé pour l'écoulement du courant du dispositif à stockage de charge vers les électrodes.

29. Circuit selon la revendication 28, dans lequel l'au moins un commutateur comprend une pluralité de commutateurs agencés sous la forme d'un pont en H (48).

30. Circuit selon l'une quelconque des revendications 14 à 29, dans lequel le circuit de commande comprend un microprocesseur (46).

31. Circuit selon l'une quelconque des revendications 14 à 30, dans lequel le circuit de commande est câblé par fil métallique.

32. Circuit selon la revendication 14, dans lequel le circuit à résistance (50) comprend une pluralité de résistances distinctes (52, 54, 56) et dans lequel le circuit de commande fournit sélectivement au moins un cheminement pour l'écoulement du courant électrique, en partant du dispositif à stockage de charge (20) et passant par un sous-ensemble de la pluralité de résistances, jusqu'à l'une des électrodes de décharge (21, 23).

33. Circuit selon la revendication 32, dans lequel le cheminement pour l'écoulement du courant électrique comprend différents sous-ensembles de résistances (52, 54, 56) pendant différentes parties de la décharge du dispositif à stockage de charge afin de produire une forme d'onde de courant rectiligne.

34. Circuit selon la revendication 33, dans lequel les résistances (52, 54, 56) sont connectées ensemble en série, le circuit comprenant des moyens (58, 60, 62) pour court-circuiter des résistances non présentes dans le sous-ensemble traversé par le cheminement.

35. Circuit selon l'une quelconque des revendications précédentes comprenant une impédance variable (66) connectée entre le dispositif à stockage de charge (20) et l'une des électrodes ; un capteur (64) qui détecte un paramètre électrique dépendant d'un patient ; et un circuit de commande connecté au capteur et à l'impédance variable, ce circuit commandant l'impédance de l'impédance variable pendant la décharge du dispositif à stockage de charge en fonction du paramètre électrique dépendant du patient détecté par le capteur.

36. Circuit selon la revendication 35, dans lequel le paramètre électrique dépendant du patient comprend l'impédance du patient.

37. Circuit selon la revendication 35 ou la revendication 36, dans lequel le circuit de commande commande l'impédance variable (66) de manière à réduire la dépendance du courant de décharge maximal par rapport au paramètre électrique dépendant du patient.

38. Circuit selon la revendication 37, dans lequel le circuit de commande sélectionne l'impédance de l'impédance variable (66) inversement par rapport à l'impédance du patient telle que détectée par le capteur.

39. Circuit selon la revendication 38, dans lequel le courant de décharge maximal, si l'impédance du patient est de 15 ohms, n'est pas supérieur à environ deux fois le courant de décharge maximal si l'impédance du patient est de 125 ohms.

40. Circuit selon la revendication 37, dans lequel la décharge du dispositif à stockage de charge (20) comprend une forme d'onde de courant ayant une partie de détection par impulsions pendant laquelle le capteur (64) détecte l'impédance du patient entre les électrodes (21, 23), et une partie de décharge thérapeutique pendant laquelle le circuit de commande sélectionne l'impédance (66) de l'impédance variable inversement par rapport à l'impédance du patient telle que détectée par le capteur (64).

41. Circuit selon la revendication 40, dans lequel la partie de décharge thérapeutique de la forme d'onde de courant est rectiligne.

42. Circuit selon l'une quelconque des revendications 35 à 41, dans lequel le capteur est un capteur de courant.

43. Circuit selon la revendication 42, dans lequel le capteur (64) est un transformateur du sens de courant.

44. Circuit selon l'une quelconque des revendications 35 à 43, dans lequel l'impédance variable (66) comprend un circuit à résistance variable.

45. Circuit selon l'une quelconque des revendications 1 à 34 comprenant un capteur (64) pour détecter un paramètre électrique dépendant d'un patient ; et un circuit de commande, connecté au capteur et au dispositif à stockage de charge (20), ce circuit commandant la décharge du dispositif à stockage de charge au travers des électrodes (21, 23), en fonction du paramètre électrique dépendant du patient tel que détecté par le capteur, de manière à réduire la dépendance du courant de décharge maximal par rapport au paramètre électrique pour un montant donné de la charge stockée par le dispositif à stockage de charge.

46. Circuit selon la revendication 45 comprenant une impédance variable (66) connectée entre le dispositif à stockage de charge et l'une des électrodes (21, 23), le circuit de commande étant connecté à l'impédance variable et commandant la décharge du dispositif à stockage de charge en commandant l'impédance variable pendant la décharge du dispositif à stockage de charge.

47. Circuit selon l'une quelconque des revendications 1 à 34 comprenant un capteur (64) pour détecter un paramètre électrique dépendant d'un patient ; et un circuit de commande, connecté au capteur et au dispositif à stockage de charge (20), ce circuit commandant la décharge du dispositif à stockage de charge au travers des électrodes (21, 23), la décharge du dispositif à stockage de charge comprenant une forme d'onde de courant ayant une partie de détection par impulsions, avec une énergie insuffisante pour exécuter la thérapie, pendant laquelle le capteur (64) détecte un paramètre électrique dépendant du patient, et une partie de décharge thérapeutique, avec une énergie suffisante pour exécuter la thérapie, ayant un courant de décharge initial commandé par le circuit de commande en fonction du paramètre électrique dépendant du patient tel que détecté par le capteur, la partie de détection par impulsions ayant un courant de décharge qui représente au moins un tiers environ du courant de décharge initial de la partie de décharge thérapeutique.

48. Circuit selon la revendication 47, dans lequel la partie de détection par impulsions a un courant de décharge qui représente au moins la moitié environ du courant de décharge initial de la partie de décharge thérapeutique.

49. Circuit selon la revendication 47 ou la revendication 48, dans lequel la partie de détection par impulsions a un courant de décharge non-alternatif.

50. Circuit selon l'une quelconque des revendications 47 à 49, dans lequel la décharge du dispositif à stockage de charge (20) se produit sans recharger le dispositif à stockage de charge entre la partie de détection par impulsions et la partie de décharge thérapeutique de la forme d'onde de courant.

51. Circuit selon l'une quelconque des revendications 47 à 50, dans lequel le circuit de commande commande la décharge du dispositif à stockage de charge (20) au travers des électrodes (21, 23), en fonction du paramètre électrique dépendant du patient tel que détecté par le capteur (64), de manière à réduire la dépendance du courant de décharge maximal par rapport au paramètre électrique dépendant du patient pendant la partie de décharge thérapeutique de la forme d'onde de courant.

52. Circuit selon l'une quelconque des revendications 1 à 34 comprenant un capteur (64) pour détecter un paramètre électrique dépendant d'un patient ; et un circuit de commande, connecté au capteur et au dispositif à stockage de charge (20), ce circuit commandant la décharge du dispositif à stockage de charge au travers des électrodes (21, 23), la décharge du dispositif à stockage de charge comprenant une forme d'onde de courant ayant une partie de détection par impulsions, avec une énergie insuffisante pour exécuter la thérapie, pendant laquelle le capteur détecte un paramètre électrique dépendant du patient, et une partie de décharge thérapeutique, avec une énergie suffisante pour exécuter la thérapie, ayant un courant de décharge initial commandé par le circuit de commande en fonction du paramètre électrique dépendant du patient tel que détecté par le capteur pendant la partie de détection par impulsions, la décharge du dispositif à stockage de charge se produisant sans recharger le dispositif à stockage de charge entre la partie de détection par impulsions et la partie de décharge thérapeutique de la forme d'onde de courant.

53. Circuit selon la revendication 52, dans lequel la partie de détection par impulsions est intégrée à la partie de décharge thérapeutique.

54. Circuit selon l'une quelconque des revendications 1 à 34 comprenant un capteur (64) pour détecter un paramètre électrique dépendant d'un patient pendant une partie de détection par impulsions d'une forme d'onde de courant, la partie de détection par impulsions ayant une énergie insuffisante pour exécuter la thérapie ; et un circuit de commande, connecté au capteur (64) et au dispositif à stockage de charge (20) pour commander la décharge du dispositif à stockage de charge au travers des électrodes (21, 23), la décharge du dispositif à stockage de charge comprenant la forme d'onde de courant ayant la partie de détection par impulsions pendant laquelle le capteur détecte un paramètre électrique dépendant du patient, la forme d'onde de courant ayant également une partie de décharge thérapeutique, avec une énergie suffisante pour exécuter la thérapie, ayant un courant de décharge initial commandé par le circuit de commande en fonction du paramètre électrique dépendant du patient tel que détecté par le capteur, la partie de détection par impulsions de la forme d'onde de courant étant intégrée à la partie de décharge thérapeutique de la forme d'onde de courant.

55. Circuit selon l'une quelconque des revendications précédentes, dans lequel les électrodes (21, 23) sont des électrodes non-implantées.

56. Circuit selon l'une quelconque des revendications précédentes, dans lequel les électrodes sont des électrodes transthoraciques.

57. Circuit selon l'une quelconque des revendications précédentes, dans lequel les électrodes (21, 23) sont adaptées à une application externe sur le patient.

58. Circuit selon la revendication 57, dans lequel les électrodes (21, 23) sont adaptées pour être appliquées au coeur d'un patient pendant une intervention chirurgicale.

59. Circuit selon l'une quelconque des revendications précédentes adapté pour appliquer le courant comme une forme d'onde de défibrillation.

60. Circuit selon l'une quelconque des revendications précédentes, dans lequel le dispositif à stockage de charge (20) comprend au moins un condensateur.

61. Circuit selon la revendication 60, dans lequel le dispositif à stockage de charge (20) est un condensateur unique.
